(19) Europäisches Patentamt
European Patent Office
Office européen des brevets

(11) Numéro de publication : 0 308 328 B1

# (12) FASCICULE DE BREVET EUROPEEN

(45) Date de publication du fascicule du brevet : **04.12.91 Bulletin 91/49**

(51) Int. Cl.[5] : **C07D 211/14,** A61K 31/445

(21) Numéro de dépôt : **88402320.1**

(22) Date de dépôt : **15.09.88**

(54) **Dérivés de (benzyl pipéridino)-1 propanol-2, leur préparation, leur utilisation comme antimicrobiens et les produits les contenant.**

(30) Priorité : **17.09.87 FR 8712885**

(43) Date de publication de la demande : **22.03.89 Bulletin 89/12**

(45) Mention de la délivrance du brevet : **04.12.91 Bulletin 91/49**

(84) Etats contractants désignés : **AT BE CH DE ES FR GB GR IT LI LU NL SE**

(56) Documents cités :
**FR-A- 1 548 392**
**FR-A- 2 201 087**
**FR-A- 2 465 732**
**US-A- 3 912 733**

(73) Titulaire : **SANOFI**
**40, Avenue George V**
**F-75008 Paris (FR)**

(72) Inventeur : **van Broeck, Didier**
**367, Avenue du Champ des Moulins**
**F-34570 Murviel les Montpellier p/Pignan (FR)**
Inventeur : **Mosse, Madeleine**
**La Chanterelle Chemin du Réservoir du Montmaur**
**F-34100 Montpellier (FR)**

(74) Mandataire : **Gillard, Marie-Louise et al**
**Cabinet Beau de Loménie 55, Rue d'Amsterdam**
**F-75008 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux dérivés de (benzyl pipéridino)-1 propanol-2.

La présente invention concerne également l'utilisation des composés selon l'invention dans des compositions à usage antiseptique, antimicrobien, antifongique, comme désinfectants ou conservateurs, notamment dans les domaines de la pharmacie, la cosmétologie ou l'agroalimentaire.

Par un autre de ses aspects, la présente invention se réfère au procédé de préparation des composés selon l'invention.

De nombreux dérivés amino-1 aryloxy-3 propanol-2 sont connus et ont été décrits pour leurs activités cardiovasculaires, notamment leurs propriétés bêta-adrénergiques. Pour illustrer cette série chimique, on peut, par exemple, citer le propranolol qui répond à la formule suivante :

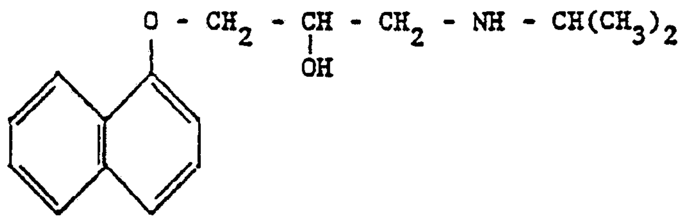

Par ailleurs, des dérivés pipéridino-1 aryloxy-3 propanol-2 ont été décrits.

Le brevet français publié sous le n° 1548392 décrit des composés de formule :

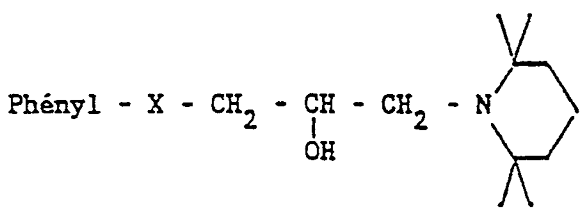

dans laquelle X = O ou S
et le radical phényle porte éventuellement un substituant.

Le brevet allemand publié sous le n° 2348898 décrit des composés de formule :

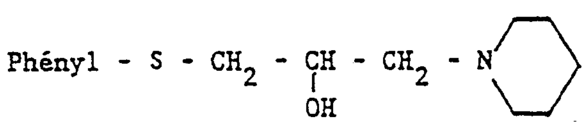

L'article japonais de IGARASHI HARUYOSHI et al., Shika Kiso Igakkai Zasshi, 1983, 25 (4), 867-74, (Chemical Abstracts 100 167 725) décrit des composés de formule :

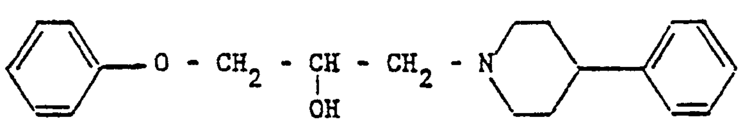

Selon la présente invention, il a été trouvé de nouveaux dérivés (benzyl pipéridino)-1 propanol-2 qui présentent des propriétés antimicrobiennes.

Ainsi, la présente invention a pour objet de nouveaux dérivés de (benzyl pipéridino)-1 propanol-2 de formule :

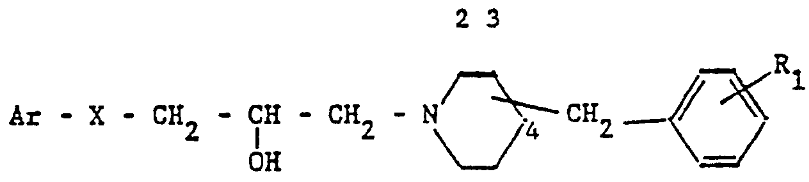

(I)

dans laquelle :

— Ar représente un groupement phényle substitué par $R_2$, $R_3$ et $R_4$ ou un groupement naphtyle-1 ou naphtyle-2, lesdits groupements naphtyles étant non substitués ou substitués par 1 ou 2 atomes d'halogène ;

— X représente un atome d'oxygène ou un atome de soufre ;

— $R_1$ représente un atome d'hydrogène ou un atome d'halogène ;

— $R_2$ représente un atome d'halogène, un groupement trifluorométhyle, un groupement phényle non substitué ou substitué par 1 à 3 atomes d'halogène, un groupement benzyle non substitué ou substitué par 1 à 3 atomes d'halogène, un groupement phénoxy non substitué ou substitué par 1 à 3 atomes d'halogène, ou un groupe alkyle contenant de 1 à 4 atomes de carbone ;

— $R_3$ et $R_4$ représentent l'hydrogène, un atome d'halogène ou un groupe alkyle contenant de 1 à 4 atomes de carbone ;

— le groupement benzyle substitue le radical pipéridino en position 2, 3 ou 4

ainsi que leurs sels avec acides minéraux ou organiques comme l'acide chlorhydrique, l'acide phosphorique, l'acide sulfurique, les acides alkylsulfoniques tels que l'acide méthane sulfonique ou l'acide lauryl sulfonique, l'acide iséthionique.

Les composés (I) contiennent un carbone asymétrique ; la présente invention comprend chacun des énantiomères des composés (I) ainsi que les racémiques.

La présente invention a également pour objet le procédé de préparation des composés de formule (I), procédé caractérisé en ce que l'on traite l'époxyde de formule :

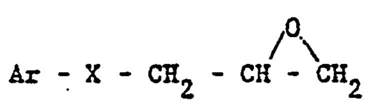

(II)

dans laquelle Ar et X ont les significations indiqués ci-dessus par la benzyl pipéridine de formule :

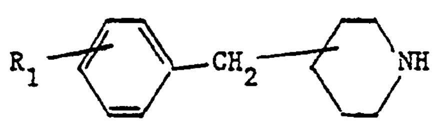

(III)

dans laquelle $R_1$ a la signification indiquée ci-dessus et le groupe benzyle substitue la pipéridine en position 2, 3 ou 4, dans un solvant protique, à une température comprise entre la température ambiante et la température d'ébullition du solvant ; et l'on isole le composé (I) sous forme d'une base libre ou sous forme d'un sel avec un acide minéral ou organique.

La réaction est effectuée avantageusement avec une quantité équimoléculaire des 2 réactifs ou en présence d'un excès de l'époxyde (II).

Comme solvant protique, on peut utiliser par exemple l'eau ou un alcool primaire comme le méthanol, l'éthanol, l'isopropanol ou méthoxyéthanol.

La réaction de formation de (I) est complète après un temps compris entre 1 heure et 48 heures selon les conditions opératoires.

Les époxydes (II) sont connus ou sont préparés par des méthodes connues. Ainsi, on peut préparer (II) en traitant un composé de formule :

Ar-X-H (IV)

dans laquelle Ar et X ont les significations indiquées ci-dessus par le chloro-1 époxy-2,3 propane ou le bromo-1 époxy-2,3 propane, dans un solvant en milieu basique.

On peut, par exemple, préparer un époxyde (II) par action du chloro-1 époxy-2,3 propane en excès sur un composé (IV) en solution dans un solvant cétonique comme la butanone en présence de carbonate de potassium.

Les benzyl pipéridines (III) sont connues ou préparées par des méthodes connues. Ainsi, on peut préparer une benzylpipéridine (III) à partir de la benzylpyridine correspondante par hydrogénation catalytique.

Les exemples suivants illustrent l'invention sans toutefois la limiter.

EXEMPLE 1

(Benzyl-4 pipéridino)-1 (dichloro-2,4 phénoxy)-3 propanol-2.

SR 44333.

A) (Dichloro-2,4 phénoxy)-3 époxy-1,2 propane

On porte à reflux pendant 10 heures un mélange contenant 81,5 g de dichloro-2,4 phénol dans 120 g de chloro-1 époxy-2,3 propane et 138 g de carbonate de potassium.

Les sels minéraux formés sont filtrés et essorés. Le filtrat limpide est évaporé sous vide et le résidu huileux est distillé sous 0,01 mm de mercure. On obtient le produit attendu sous forme d'une huile incolore.

| | |
|---|---|
| Point d'ébullition : | 124-126°C |
| Poids : | 82 g |
| Rendement : | 75% |

B)SR 44333

On laisse sous agitation pendant 48 heures à température ambiante une solution contenant 8,75 g de benzyl-4 pipéridine et 11,5 g d'époxyde préparé à l'étape précédente dans 100 ml de méthanol.

Il se forme une solution limpide incolore que l'on évapore à demi ce qui provoque la cristallisation, on refroidit dans la glace pour compléter la cristallisation, puis essore les cristaux blancs formés, lave rapidement avec du méthanol glacé et sèche sous vide.
On obtient 12,65 g du produit attendu.

| | |
|---|---|
| Rendement : | 64% |
| Point de fusion : | 88-90°C |

| Analyse élémentaire : | C | H | N | Cl |
|---|---|---|---|---|
| calculé | 63,96 | 6,39 | 3,55 | 17,98 |
| trouvé | 64,10 | 6,54 | 3,59 | 17,76 |

C) Méthane sulfonate de (benzyl-4 pipéridino)-1 (dichloro-2,4 phénoxy)-3 propanol-2.

SR 44333 B

On dissout 10 g du SR 44333 dans 200 ml d'éther en tiédissant la solution et l'on ajoute lentement 2,44 g d'acide méthane sulfonique dans 50 ml d'éther, le sel concrétise. On agite 2 heures puis essore les cristaux blancs, lave à l'éther et sèche sous vide.

| | |
|---|---|
| Poids : | 12,25 g |
| Rendement : | 98,6% |
| Point de fusion : | 116-118°C |

| Analyse élémentaire : | C | H | N | Cl |
|---|---|---|---|---|
| calculé | 53,88 | 5,96 | 2,85 | 14,46 |
| trouvé | 54,03 | 6,10 | 2,70 | 14,46 |

D) Chlorhydrate de (benzyl-4 pipéridino)-1 (dichloro-2,4 phénoxy)-3 propanol-2

SR 44333 A

On dissout 12,8 g de base libre dans 150 ml d'acétone. On ajoute goutte à goutte une quantité suffisante d'acide chlorhydrique gazeux en solution dans l'éther. Le sel cristallise rapidement sous forme de cristaux blancs.

Poids obtenu : 12,05 g
Rendement : 93,3%
Point de fusion : 144-146°C

| Analyse élémentaire : | C | H | N | Cl |
|---|---|---|---|---|
| calculé | 58,55 | 6,08 | 3,25 | 24,69 |
| trouvé | 58,68 | 6,21 | 3,16 | 24,54 |

EXEMPLE 2

Chlorhydrate de [(benzyl-2 chloro-4) phénoxy]-3 (benzyl-4 pipéridino)-1 propanol-2.

SR 44719 A

A) [(Benzyl-2 chloro-4) phénoxy]-3 époxy-1,2 propane.

On porte à reflux pendant 12 heures une suspension contenant 21,87 g de (benzyl-2 chloro-4) phénol, 27,76 g de chloro-1 époxy-2,3 propane et 27,64 g de carbonate de potassium dans 200 ml de butanone. Les sels minéraux formés sont filtrés et essorés. Le solvant et l'excès de chloro-1 époxy-2,3 propane sont évaporés sous vide et le résidu est distillé entre 160 C et 180°C sous 0,01 mm de mercure.

L'huile incolore obtenue se solidifie rapidement.

Poids : 21,7 g
Rendement : 79%

B) SR 44719 A

On laisse sous agitation à température ambiante pendant 48 heures une solution contenant 11,6 g de l'époxyde obtenu à l'étape précédente et 7 g de benzyl-4 pipéridine dans 100 ml de méthanol. Le méthanol est chassé sous vide, puis le résidu est repris avec 200 ml d'acétone et traité par une quantité suffisante d'acide chlorhydrique gazeux en solution dans l'éther. Les cristaux blancs formés sont essorés, lavés par un mélange acétone-éther et séchés sous vide.

Poids : 16,3 g
Rendement : 83,8%
Point de fusion : 139-141°C

| Analyse élémentaire : | C | H | N |
|---|---|---|---|
| calculé | 69,13 | 6,84 | 2,88 |
| trouvé | 68,98 | 7,06 | 2,78 |

EXEMPLE 3

Chlorhydrate de (benzyl-4-pipéridino)-1 (bromo-4 phénylthio)-3 propanol-2

SR 44444 A

A) (Bromo-4 phénylthio)-3 époxy-2,3 propane

On porte à reflux pendant 12 heures une suspension contenant 9,45 g de bromo-4 thiophénol, 14,45 g de chloro-1 époxy-2,3 propane et 13,8 g de carbonate de potassium dans 100 ml de butanone. On filtre et essore les sels minéraux formés puis on évapore le solvant et l'excès de chloro-1 époxy-2,3 propane sous vide. L'huile résiduelle est distillée sous 0,01 mm de mercure entre 130°C et 140°C.

Poids obtenu : 10,43 g
Rendement : 85%

B) SR 44444 A

Ce produit est préparé en opérant comme dans les exemples précédents à partir de 2,6 g d'époxyde obtenu à l'étape A et 1,75 g de benzyl-4 pipéridine.

Le chlorhydrate formé est cristallisé dans l'acétone.

Poids obtenu : 4,41 g
Rendement : 97%
Point de fusion : 158-160°C

| Analyse élémentaire : | C | H | N |
|---|---|---|---|
| calculé | 55,21 | 5,96 | 3,06 |
| trouvé | 55,29 | 6,04 | 2,94 |

EXEMPLE 4

Chlorhydrate de (benzyl-4 pipéridino)-1 (bromo-1 naphtyl-2-oxy)-3 propanol-2

SR 44716 A

A) (Bromo-1 naphtyl-2-oxy)-3 époxy-1,2 propane

On porte à reflux pendant une nuit une suspension contenant 22,3 g de bromo-1 naphtol-2, 27,75 g de chloro-1 époxy-2,3 propane et 27,6 g de carbonate de potassium dans 200 ml de butanone. On filtre et essore les sels minéraux puis on évapore sous vide. L'huile obtenue est distillée sous 0,01 mm de mercure.

Poids obtenu : 22,63 g
Rendement : 81%
Température d'ébullition : 165-185°C

B) SR 44716 A

En opérant comme dans les exemples précédents à partir de 2,93 g d'époxyde obtenu à l'étape A et 1,75 g de benzyl-4 pipéridine dans 25 ml de méthanol, on obtient le produit attendu qui après salification cristallise dans l'acétone sous forme de cristaux blancs.

Poids : 3,99 g
Rendement : 81,3%
Point de fusion : 159-161°C

| Analyse élémentaire : | | C | H | N |
|---|---|---|---|---|
| | calculé | 61,17 | 5,75 | 2,85 |
| | trouvé | 61,36 | 6,00 | 2,74 |

EXEMPLE 5

Méthane sulfonate de (benzyl-3 pipéridino)-1 (dichloro-2,4 phénoxy)-3 propanol-2 :

SR 44957 A

A) (Dichloro-2,4 phénoxy)-3 époxy-1,2 propane

Ce produit est préparé à l'exemple 1, étape A.

B) (para-chlorobenzyl)-4 pipéridine.

Ce produit est préparé en suivant la méthode décrite dans le brevet US 3682 767.

0,2 ml de (para-chlorobenzyl)-4 pyridine sont dissous dans 500 ml d'acide citrique, on ajoute 3,7 g d'oxyde de platine et on place le mélange dans un autoclave sous une pression d'hydrogène de 4 bars, on laisse sous agitation pendant 7 heures. On ajoute 0,5 g d'oxyde de platine et place pendant 5 heures sous atmosphère d'hydrogène.

Après filtration du catalyseur, le filtrat est concentré à sec sous vide, le résidu est repris dans l'eau glacée puis on basifie (pH = 10-11) par addition d'une solution de soude à 10% puis d'une solution de soude à 30%.

Après extraction à l'acétate d'éthyle et séchage sur sulfate de magnésium, on obtient une huile qui cristallise par addition d'acide chlorhydrique dans l'éther.

Poids obtenu : 43,4 g
Rendement : 88%

C) SR 44957 A

Ce produit est préparé selon la méthode décrite dans les exemples précédents.

Le méthane sulfonate cristallise dans l'éther.

Point de fusion : 114-116°C

En opérant de la même manière, on a préparé les composés selon l'invention qui sont décrits dans le tableau I ci-dessous.

TABLEAU I

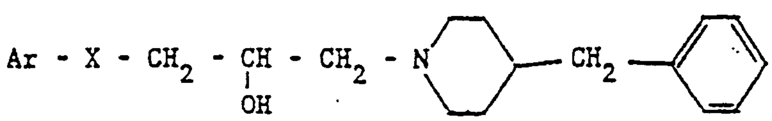

| Numéro de produit (n°exemple) | Ar | X | Sel Point de fusion | (solvant de formation) |
|---|---|---|---|---|
| SR 44251 A (6) | bromo-4 phényl | O | chlorhydrate 176-178°C | (éther) |
| SR 44334 A (7) | chloro-4 phényl | O | chlorhydrate 146-148°C | (acétone) |
| SR 44473 A (8) | dichloro-3,4 phényl | S | chlorhydrate 150-152°C | (acétone) |
| SR 44560 A (9) | trichloro-2,4,5 phényl | O | chlorhydrate 144-146°C | (acétone/ éther) |
| SR 44641 A (10) | chloro-5 (dichloro-2,4 phénoxy)-2 phényl | O | chlorhydrate 134-136°C | (éther) |
| SR 44644 A (11) | naphtyl-1 | O | chlorhydrate 199-201°C | (acétone) |
| SR 44644 B (12) | " | O | méthanesulfonate 141-143°C | (acétone) |
| SR 44648 A (13) | tertiobutyl-4 phényl | O | chlorhydrate 181-183°C | (acétone) |
| SR 44674 A (14) | (bromo-4 naphtyl-2) | O | chlorhydrate 190-192°C | (acétone) |

| | | | | |
|---|---|---|---|---|
| SR 44676 A (15) | (phényl-2) phényl | O | chlorhydrate 139-141°C | (acétone) |
| SR 44715 A (16) | (phényl-4) phényl | O | chlorhydrate 177-179°C | (acétone) |
| SR 44715 B (17) | " | O | méthanesulfonate 152-154°C | (acétone) |
| SR 44719 B (18) | benzyl-2 chloro-4 phényl | O | méthanesulfonate 124-126°C | (éther) |
| SR 44807 A (19) | naphtyl-2 | O | méthane sulfonate 135-137°C | (acétone/ éther) |
| SR 44971 A (20) | difluoro-2,4 phényl | O | chlorhydrate 123-125°C | (acétone/ éther) |
| SR 44999 A (21) ** | naphtyl-1 | O | méthane sulfonate 181-183°C | (acétone/ éther) |
| SR 45076 A (22) ** | bromo-4 phényl | S | chlorhydrate 167-169°C | (acétone) |
| SR 45077 A (23) ** | dichloro-3,4 phényl | S | chlorhydrate 134-136°C | (acétone) |
| SR 45204 A (24) | bromo-3 phényl | S | chlorhydrate 129-131°C | (acétone) |

| | | | | | |
|---|---|---|---|---|---|
| | | | chlorhydrate | | |
| (SR 45236 A | dichloro-3,4 | S | 129-131°C | (acétone) | |
| (SR 45237 A | phényl | | 113-115°C | (acétone/ | |
| (25 & 26)* | | | | éther) | |
| | | | | | |
| SR 45551 A | dichloro-3,4 | O | chlorhydrate | (acétone/ | |
| (27) | phényl | | 151-153°C | éther) | |
| | | | | | |
| SR 45553 A | dichloro-3,4 | O | chlorhydrate | (acétone | |
| (28) ** | phényl | | 139-140°C | éther) | |

* Le groupe benzyl substitue le radical pipéridino en position 3. Les SR 45236 A et SR 45237 A sont les 2 diastéreoisomères séparés par chromatographie sur colonne de silice.

** Les composés des exemples 21; 22; 23 et 28 portent un substituant $R_1$ = para-chloro sur le benzyle.

Les activités bactéricides et fongicides des produits selon l'invention ont été étudiées sur différentes souches.

Un inoculum bactérien est mis en contact avec différentes dilutions du produit à tester, et ce pendant un temps limité : 30 minutes. A la fin du contact, une partie aliquote du mélange suspension bactérienne/produit est déposée à la surface d'un milieu de culture gélosé contenant un neutralisant de l'activité antibactérienne du produit. La concentration bactéricide retenue est la concentration minimale du produit à partir de laquelle les bactéries ne poussent plus. Cette concentration est exprimée en µg/ml.

Les souches bactériennes choisies pour l'étude sont :

1 — Staphylococcus aureus CNCM 53154 ;
2 — Streptococcus faecalis CNCM 5855 ;
3 — Pseudomonas aeruginosa CNCM A22 ;
4 — Escherichia coli CNCM 54127.

Les souches sont entretenues sur Tryptic Soy Agar (TSA), commercialisé par Difco.

Après 24 heures de culture à 37°C, on récolte la pousse microbienne à l'aide de billes de verre et de 10 ml de diluant contenant 1 g de tryptone et 8,5 g de chlorure de sodium dans 100 ml d'eau distillée. On agite la suspension formée et on mesure au spectrophotomètre le pourcentage de transmission de la luminère à 620 nm :

— Souche 1 : 60% ;
— Souche 2 : 60% ;
— Souche 3 : 70% ;
— Souche 4 : 70%.

L'inoculum bactérien correspond à une dilution au 1/20 ème de cette suspension bactérienne.

Des plaques comportant des cupules reçoivent différentes dilutions du produit à étudier. Ces dilutions du produit à étudier sont mises en contact avec les différentes suspensions bactériennes à l'aide d'un inoculateur à sites multiples. Après 30 minutes de contact, des parties aliquotes sont transférées à l'aide de cet inoculateur à la surface d'un milieu gélosé (TSA) placé dans des boîtes de Pétri, contenant un neutralisant de l'activité, à savoir 20 g de lubrol W, 2,5 g de Tween 80, 2,5 g de thiosulfate de sodium et 1% de jaune d'oeuf dans 1000 ml de TAS (Difco). Un témoin de l'efficacité du neutralisant est réalisé pour chaque produit étudié en déposant à la surface du milieu de culture une partie aliquote de la dilution du produit à étudier. Après séchage, l'inoculum correspondant est déposé au même endroit. Un témoin inoculum est réalisé sur milieu gélosé avecet sans neutralisant. La lecture se fait après 48 heures d'incubation à 37°C.

L'activité antifongique des produits selon l'invention a également été déterminée en utilisant la méthode décrite précédemment. Une souche représentative des levures a été choisie pour l'étude : Candida albicans CNCM 1180 (souche 5).

Elle est entretenue sur milieu gélosé de Sabouraud Dextrose Agar, commercialisé par Difco. La technique est identique à celle décrite pour l'étude de l'activité antibactérienne. Après 48 heures de culture à 37°C, on récolte la pousse microbienne à l'aide de billes de verre et de 5 ml de diluant contenant 1 g de tryptone et 8,5 g de chlorure de sodium dans 1000 ml d'eau distillée ; 5 ml du diluant sont ensuite rajoutés. Cette suspension donne au spectrophotomètre un pourcentage de transmission de la luminère à 620 nm de 2 à 3%. L'inoculum correspond à une dilution au 1/10 de cette suspension microbienne. Une dilution au 1/100 de cette suspension, observée entre lame et lamelle à l'objectif 40 d'un microscope, doit montrer 10 cellules par champ, ce qui correspond à 1.000.000 levures par ml.

Les résultats sont rassemblés dans le tableau II ci-dessous. Ils indiquent les concentrations minimales bactéricides (CMB) pour les souches 1, 2, 3, 4 et les concentrations minimales fongicides (CMF) pour la souche 5.

A titre de comparaison on a préparé des dérivés aryloxy-3 pipéridino-1 propanol-2 décrits dans la littérature ou proches des composés décrits et l'on a mesuré pour ces composés ainsi que pour le propranolol les activités bactéricides et fongicides sur les mêmes souches et dans les mêmes conditions que pour les produits selon l'invention. Les résultats sont reportés dans la tableau II à la suite de ceux obtenus avec les produits selon l'invention.

Les produits de comparaison sont les suivants :

— Produit A : chlorhydrate de (bromo-4 phényloxy)-3 (méthyl-4 pipéridino)-1 propanol-2
Point de fusion : 194-196°C

— Produit B : chlorhydrate de phényloxy-3 (phényl-4 pipéridino)-1 propanol-2
Point de fusion : 151-153°C

— Produit C : chlorhydrate de (bromo-4 phénylthio)-3 pipéridino-1 propanol-2.
Point de fusion : 152-154°C

— Propranolol

TABLEAU II

CMB - CMF en µg/ml

| N° de produit (Agent solubilisant) | Souches 1 | 2 | 3 | 4 | 5 |
|---|---|---|---|---|---|
| SR 44251 A (eau) | 100 | 100 | 100 | 100 | - |
| SR 44333 A (TEG) | 100 | 100 | 50 | 50 | - |
| SR 44333 B (eau) | 50 | 50 | 50 | 50 | 50 |
| SR 44334 A (TEG) | 500 | 100 | 100 | 100 | - |
| SR 44444 A (TEG) | 50 | 50 | 50 | 50 | - |
| SR 44473 A (TEG) | 1000 | 50 | 50 | 50 | 50 |
| SR 44560 A (TEG) | 10 | 50 | 50 | 5 | 50 |
| SR 44641 A (acétone) | 50 | 10 | 10 | 5 | 50 |

| | | | | | |
|---|---|---|---|---|---|
| SR 44644 A (TEG) | 50 | 50 | 50 | 50 | 50 |
| SR 44648 A (acétone) | 200 | 200 | 50 | 50 | 200 |
| SR 44674 A (acétone) | 10 | 10 | 10 | 10 | 200 |
| SR 44676 A (TEG) | 200 | 50 | 10 | 10 | 50 |
| SR 44715 A (TEG) | 50 | 50 | 50 | 50 | 200 |
| SR 44716 A (TEG) | 50 | 50 | 10 | 10 | 50 |
| SR 44719 A (acétone) | 10 | 10 | 10 | 5 | 50 |
| SR 44719 B (TEG) | 10 | 5 | 10 | 5 | 10 |
| SR 44807 A (eau) | 1000 | 1000 | 200 | 200 | 1000 |
| SR 44957 A (TEG) | 50 | $\leqslant$ 10 | $\leqslant$ 10 | $\leqslant$ 10 | 50 |
| SR 44971 A (TEG) | 1000 | 1000 | 1000 | 1000 | 1000 |

| | | | | | |
|---|---|---|---|---|---|
| SR 44999 A (TEG) | 50 | ≤ 10 | ≤ 10 | ≤ 10 | ≤ 10 |
| SR 45076 A (TEG) | 50 | 10 | 50 | 10 | 50 |
| SR 45077 A (TEG) | 50 | 10 | 50 | 10 | 50 |
| SR 45204 A (TEG) | > 200 | > 200 | 50 | 50 | > 200 |
| SR 45236 A (TEG) | > 200 | 50 | 50 | 50 | > 200 |
| SR 45237 A (TEG) | > 200 | 50 | 50 | 50 | > 200 |
| SR 45551 A (TEG) | > 200 | 50 | 50 | 50 | > 200 |
| SR 45552 A (TEG) | > 200 | 50 | 50 | 10 | 50 |
| SR 45553 A (TEG) | 10 | 50 | 50 | 10 | 50 |
| Produit A (eau) | >2000 | 1000 | 1000 | 1000 | 5000 |
| Produit B (eau) | >1000 | >1000 | >1000 | >1000 | >1000 |

| | | | | | |
|---|---|---|---|---|---|
| Produit C (eau) | >1000 | >1000 | >1000 | >1000 | 1000 |
| Propranolol (eau) | 5000 | 1000 | 1000 | 1000 | 5000 |
| L'abréviation TEG signifie tétraéthylèneglycol à 20 % dans l'eau. | | | | | |

Les résultats montrent que les produits selon l'invention présentent un large spectre d'activité sur les souches bactériennes et fongique testées.

Cette activité s'exprime dans un temps court (inférieur ou égal à 30 minutes).

Au contraire, les produits A, B, C et le propranolol ne présentent aucune activité antimicrobienne.

Par ailleurs, il a été recherché une éventuelle activité des composés selon l'invention sur le système cardiovasculaire.

En particulier, on a étudié l'affinité de ces composés pour les récepteurs β adrénergiques par une technique biochimique.

La méthode utilisée est celle décrite par R.W. Alexander, L.T. Williams et R.J. Leftkowitz, Proc. Nat. Acad. Sci. USA, 1975, 72 (4), 1564-1568.

Sur une préparation d'homogénats de membrane de coeur de chien et de poumon de rat qui comprennent des récepteurs $\beta_1$ et $\beta_2$ adrénergiques, on mesure l'affinité pour les sites de liaison des produits à étudier par compétition avec un ligand radioactif : le dihydroalprénolol tritié.

On a observé que les composés selon l'invention présentent une affinité aux récepteurs adrénergiques 1000 à 10000 fois inférieure à celle du propranolol.

Il apparait que les composés selon l'invention ne présentent aucune activité bêta-adrénergique.

Ainsi les composés selon l'invention représentent une famille de composés nouveaux qui se distinguent des composés connus tant par leur structure chimique nouvelle que par leur activité.

La tolérance des produits selon l'invention a été étudiée chez le cobaye. Les animaux sont tondus de part et d'autre de la ligne médiane du dos, la tonte est entretenue tous les 2 jours. Des lots de 6 animaux reçoivent sur la zone tondue 0,2 ml d'une solution aqueuse ou alcoolique du produit selon l'invention. Lorsque les produits sont en solution alcoolique, un lot témoin d'animaux reçoit l'alcool sur un côté.

Pour l'étude de la tolérance cutanée, le traitement est appliqué 1 fois par jour, 6 jours sur 7, durant 3 semaines. Les observations sur le plan cutané portent sur la présence d'érythème, d'éruption cutanée ou d'hyperkératose dont l'intensité est graduée selon un barème déterminé.

L'essai de sensibilisation cutanée est réalisé sur les mêmes animaux après deux semaines de repos. Le traitement dure une semaine, il est identique au précédent. L'évaluation se fait sur les mêmes critères et d'après le même barème que celui utilisé pour la tolérance locale.

On a constaté que les produits selon l'invention sont bien tolérés lorsqu'ils sont appliqués à des concentrations allant jusqu'à 2%. De plus ils ne présentent aucun effet sensibilisant.

Une évaluation de la toxicité aiguë par voie orale a été réalisée chez la souris. Cette étude a été effectuée sur des souris mâles souche CD1 provenant de l'élevage Charles River. Chaque lot était composé de 5 animaux d'un poids corporel variant de 24 à 30 g entretenus dans une même cage. Les animaux étaient conservés à jeun pendant 6 heures avant le traitement. Pour chaque étude, le produit, mis en suspension dans une solution de gomme arabique à 10%, a été administré par gavage à l'aide d'une sonde oesophagienne. La nourriture était de nouveau distribuée aux animaux 4 heures après le gavage et les animaux étaient gardés en observation pendant une période de 14 jours après l'administration. Pendant cette période, on note la mortalité dans chacun des lots mit en expérience et, lorsque cela est possible, on détermine la dose létale 50 ($DL_{50}$) en utilisant la méthode de J.T. LITCHFIELD et R.WILCOXON, J. Pharmacol. 1949, 95, 99-113. On a constaté que la $DL_{50}$ per os des produits selon l'invention est supérieure à 1000 mg/kg.

Les produits selon l'invention qui présentent une bonne activité antimicrobienne peuvent être utilisés dans des préparations pharmaceutique, désinfectante, cosmétique ou alimentaire, notamment comme antiseptiques par voie locale et générale, comme désinfectants et comme conservateurs.

En tant qu'antiseptiques à usage humain ou vétérinaire, la concentration en produit actif peut varier de 0,01% à 5% selon l'usage et la formulation choisie. Ainsi on peut préparer des solutions moussantes détergentes destinées au lavage des mains du chirurgien et du personnel soignant ou à la détersion de lésions dermatologiques telles que impétigo, pityriasis, ulcères des jambes. Des solutions moussantes détergentes servent également comme shampooings (antipelliculaire par exemple) ou pour la préparation de gels pour douches, de crèmes à raser, de lotions moussantes. On obtient des solutions moussantes contenant des produits selon l'invention en utilisant des tensioactifs amphotères, anioniques, cationiques ou non ioniques à une concentration de 0,3 à 30%, des agents humectants tels que les glycols ou des polyéthylèneglycols de 0 à 20%, des copolymères d'oxyde d'éthylène et de polypropylène de 0 à 20%, un alcool (éthanol, isopropanol, alcool benzylique) ou un polyol tel que le glycérol de 0 à 15%, ainsi que des complexants des ions $Ca^{++}$, $Mg^{++}$, des métaux lourds, des sels apportant un pouvoir tampon approprié, des agents viscosants tels que NaCl ou KCl, des polymères naturels, cellulosiques ou synthétiques tels que le polyvinylpyrrolidone, des surgraissants épaississants tels que le distéarate de polyéthylèneglycol, le mono- ou di-éthanolamide de coprah, des parfums, conservateurs, colorants.

Lorsque le produit selon l'invention est difficilement soluble dans l'eau, on pourra utiliser des microémulsions, des solutions micellaires ou toute autre phase du diagramme ternaire ou quaternaire eau/principe actif/tensioactif/cotensioactif, permettant la solubilisation dans l'eau. Ces solutions peuvent être diluées ou non, elles peuvent par exemple être distribuées à l'aide d'une vasopompe ou de gaz pulseurs liquéfiés ou non.

En utilisant les mêmes constituants à des concentrations appropriées, on peut également préparer, avec des produits selon l'invention, des solutions aqueuses simples ou sous forme de sprays destinés à l'antisepsie des champs opératoires, aux soins post-opératoires, soins des brûlés, eczémas surinfectés, érythèmes fessiers, plaies, acné, ou destinés à des déodorants.

Des solutions alcooliques simples ou sous forme de sprays contenant 20 à 80% d'alcool peuvent comporter, outre les excipients utilisés dans les solutions aqueuses, des excipients permettant de franchir les couches kératinisées de la peau et des phanères tels que Azone (commercialisé par Nelson Research) et Transcutol (commercialisé par Gattefossé). Ces solutions sont destinées à l'antisepsie de la peau avant ponctions, la préparation du champ opératoire, l'antisepsie des mains du personnel soignant, aux soins des dermatoses infectées fermées, folliculites, perionyxis ou acné.

Les produits selon l'invention peuvent être appliqués sous forme de crèmes qui contiennent certains des composés cités pour la préparation des solutions ainsi que les corps gras habituellement rencontrés dans la préparation des crèmes ou émulsions. Ces crèmes sont utilisables notamment pour la prévention de surinfections de l'érythème fessier, de l'eczéma, des mycoses ou de l'acné. Les produits selon l'invention peuvent être aussi utilisés dans des shampooings en tant qu'agent antipelliculaire.

Les produits selon l'invention peuvent également être utilisés pour le traitement ou la prévention des maladies sexuellement transmissibles sous forme d'ovules, comprimés gynécologiques ou éponges gynécologiques ou en complément de préservatifs. Les ovules peuvent contenir de 0 à 99% de triglycérides, polyéthylèneglycols de différents poids moléculaires, Tweens, polymères naturels ou synthétiques, polyols, savons. Les comprimés gynécologiques peuvent contenir des diluants tel que lactose ou cellulose, des lubrifiants tels que le stéarate de magnésium, des agents d'écoulement tels que la silice, des agents de désagrégation tel que le carboxyméthylamidon ou la cellulose.

Les produits selon l'invention peuvent être administrés sous forme de sprays avec embouts nasal et buccal dans les syndromes infectieux des voies respiratoires (rhinites, sinusites, angines, amygdalites, pharyngites) ou sous forme de gels ou de bains de bouche pour le traitement des gingivites, pyorrhées ou la prévention de la plaque dentaire, dans ce cas on pourra également utiliser des dentifrices contenant le produit selon l'invention. Les formes destinées à l'administration buccale ou nasale peuvent contenir les mêmes excipients que les solutions auxquelles on ajoute éventuellement des aromatisants pour la sphère buccale ou les constituants nécessaires à l'isotonicité pour les sprays nasaux ; les dentifrices contiennent en outre des silices colloïdales pyrogénées ou non, du carbonate de calcium, des édulcorants et des sels de fluor.

Les produits selon l'invention peuvent être utilisés dans des collyres, solutions oculaires ou pommades ophtalmiques pour le traitement des infections de l'oeil (blépharites ou conjonctivites par exemple) ou en liquide de rinçage des lentilles cornéennes. Pour préparer ces formes oculaires, on peut utiliser les mêmes constituants que ceux utilisés pour les solutions en veillant à assurer l'isotonicité du mélange.

De plus, les produits selon l'invention peuvent être administrés par voie générale chez l'homme par exemple par voie orale, sous forme de gélules, comprimés ou comprimés entériques comme antiseptiques intestinaux.

Les produits selon l'invention peuvent également être utilisés chez l'animal dans des indications telles que la prévention ou le traitement des lésions infectées ou susceptibles de se surinfecter. Les compositions pharmaceutiques sont alors similaires à celles utilisées chez l'homme, en particulier des crèmes, sprays ou solutions.

Par ailleurs, l'action léthale rapide sur les germes des produits selon l'invention permet de les utiliser comme désinfectants de surface à des concentrations pouvant varier de 0,1 à 4%. Les produits sont alors mis en oeuvre dans des préparations telles que des solutions moussantes détergentes, aqueuses ou non aqueuses, des sprays ou nébulisations. De telles préparations sont particulièrement utiles dans les domaines hospitaliers ou vétérinaires, pour les collectivités locales ou les industries agroalimentaires. Ces préparations peuvent contenir les mêmes constituants que ceux utilisés dans les formulations à usage antiseptique, on peut toutefois y adjoindre des solvants organiques divers.

Enfin, l'activité antimicrobienne de ces produits permet de les utiliser comme conservateurs dans les industries pharmaceutique, cosmétique et alimentaire. Les produits selon l'invention sont alors utilisés comme additifs aux formulations pharmaceutiques, cosmétiques ou alimentaires à des concentrations pouvant varier de 0,005 à 0,5%. On peut également utiliser ces composés comme additifs désinfectants dans les peintures.

Différentes formulations des produits selon l'invention peuvent être préparées selon l'application choisie. Les exemples suivants illustrent cette partie de l'invention.

EXEMPLE 6 :

Préparation antiseptique liquide détergente moussante.

| | | |
|---|---|---|
| SR 44 333 A | | 0,5 g |
| Paraffine sulfonate de sodium | | 15 g |
| Hydroxyde de sodium ou | | |
| acide lactique | qsp pH 5,2 | |
| Eau purifiée | qsp | 100 g. |

EXEMPLE 7 :

Soluté antiseptique alcoolique.

| | | |
|---|---|---|
| SR 44 333 A | | 0,5 g |
| Alkyldiméthylcarboxyméthylamine | | 0,5 g |
| (solution à 30 %) | | |
| Condensat d'oxyde d'éthylène | | |
| et de propylène glycol L 62 | | 1 g |
| Acide lactique ou | | |
| hydroxyde de sodium | qsp pH 6,5 | |
| Alcool éthylique à 70° | qsp | 100 g. |

EXEMPLE 8 :

Préparation antiseptique liquide détergente moussante.

| | | |
|---|---|---|
| SR 44 719 B | | 0,1 g |
| Alkyldiméthylcarboxyméthylamine (solution à 30 %) | | 15 g |
| Tétracémate disodique | | 0,1 g |
| Propylène glycol | | 20 g |
| Hydroxyde de sodium | qsp pH 5,8 | |
| Eau purifiée | qsp | 100 g. |

EXEMPLE 9 :

Collutoire.

| | | |
|---|---|---|
| SR 44 716 A | | 0,3 g |
| Alcool éthylique à 95° | | 14 g |
| Essence d'anis | | 0,00225 ml |
| Eugenol | | 0,00075 ml |
| Glycérine | | 20 ml |
| Saccharine | | 0,03 g |
| Hydroxyde de sodium soluté | qsp pH 5,5 | |
| Eau purifiée | qsp | 100 ml. |

EXEMPLE 10 :

Ovules antiseptiques destinées au traitement des maladies sexuellement transmissibles.

| | |
|---|---|
| SR 44 719 B | 500 mg |
| Mélange eutectique d'esters d'acides gras | 2,568 g. |

Comme mélange eutectique d'esters d'acides gras, on peut utiliser Suppocire A® commercialisé par Gattefossé.

EXEMPLE 11 :

Collyre.

| | | |
|---|---|---|
| SR 44 333 A | 0,5 g | |
| Chlorure de sodium | 1,4 g | |
| Eau pour préparations injectables | qsp | 100 ml. |

EXEMPLE 12 :

Comprimés entériques.

| | |
|---|---|
| SR 44 444 A | 200 mg |
| Hydroxypropylméthyl cellulose 6 cP | 6 mg |
| Lactose | 114 mg |
| Cellulose microcristalline | 60 mg |
| Carboxyméthylamidon sodique | 12 mg |
| Stéarate de magnésium | 8 mg |
| Pour un comprimé nu terminé à | 400 mg. |

Enrobage

| | |
|---|---|
| Eudragit L 100® | 0,9 mg |
| Phtalate dibutyle | 0,9 mg |
| Acétone | 14,1 mg |
| Alcool isopropylique | 14,1 mg |
| Comprimé enrobé terminé à | 430 mg. |

EXEMPLE 13 :

Spray.

| | | |
|---|---|---|
| SR 44 719 A | | 2 g |
| Ethanol à 95° | | 20 g |
| Propylène glycol | | 5 g |
| Hydroxyde de Na | qsp pH 5,5 | |
| Eau | qsp | 100 g |
| Agent pulseur | qs | |

EXEMPLE 14 :

Spray filmogène.

| | | | |
|---|---|---|---|
| SR 44 333 B | | 0,5 | g |
| Polyvinylpyrrolidone | | 2 | g |
| Résine acrylique | | 2 | g |
| Ethanol à 95° | qsp | 100 | g |
| Agent pulseur | qs | | |

EXEMPLE 15 :

Shampooing antipelliculaire :

| | | |
|---|---|---|
| lauryléther sulfate de sodium (solution à 30 %) | | 21 g. |
| diéthanolamine de coprah | | 2 g. |
| esters polyglycoliques d'alcools gras | | 8 g. |
| acétate de linalyle | | 0,2 g. |
| chlorure de sodium | | 0,7 g. |
| SR 44719 B | | 0,1 g. |
| eau | q.s.p. | 100 g. |

EXEMPLE 16 :

Un produit selon l'invention peut être utilisé comme conservateur dans une crème émulsion.

| | | | |
|---|---|---|---|
| Huile de vaseline | | 6 | g |
| Mélange d'alcool cétostéarylique et d'alcool cétostéarylique oxyéthyléné | | 9 | g |
| Phosphate monosodique anhydre | | 0,300 | g |
| Tétracémate disodique | | 0,010 | g |
| Vaseline | | 15 | g |
| SR 44 641 A | | 0,100 | g |
| Acide phosphorique | qsp pH 4,5 | | |
| Eau purifiée | qsp | 100 | g. |

EXEMPLE 17 :

Le produit selon l'invention peut être utilisé comme conservateur dans une crème pour utilisation cosmétologique.

| | |
|---|---|
| Collagène | 0,500 g |
| Carboxypolyméthylène 934 | 0,400 g |
| Lanoline hydrogénée | 4 g |
| Perhydrosqualène | 20 g |
| Monopalmitate de sorbitol polyoxyéthyléné | 2 g |
| SR 44 444 A | 0,150 g |
| Acide lactique ou hydroxyde de sodium qsp pH 6,5 | |
| Eau purifiée qsp | 100 g. |

EXEMPLE 18 :

Conservateur dans une huile antisolaire.

| | |
|---|---|
| Huile minérale 65/75 | 68 g |
| Huile de ricin | 8 g |
| Huile de sésame | 20 g |
| Alcool isopropylique | 2 g |
| Eusolex® | 1,5 g |
| Parfum | 0,4 g |
| SR 44 719 B | 0,100 g. |

(Eusolex® est commercialisé par Merck)

EXEMPLE 19 :

Un produit selon l'invention peut être utilisé comme conservateur dans un shampooing.

| | |
|---|---|
| Palmitate de potassium et d'acides aminés | 20 g |
| Alkylsulfates de sodium | 2 g |
| Diéthanolamide de coprah | 5 g |
| Acétate de linalyle | 0,200 g |
| SR 44 641 A | 0,05 g |
| Hydroxyde de sodium qsp pH 7 | |
| Eau purifiée qsp | 100 g. |

EXEMPLE 20 :

Conservateur pour jus de fruits ou confiture.

SR 44719 A micronisé 0,02%

EXEMPLE 21 :

Désinfectant pour surfaces inertes.

| | | | |
|---|---|---|---|
| SR 44 333 B | | | 2 g |
| Dodécyldiméthylcarboxydiméthylamine | | | 20 g |
| Tétracémate disodique | | | 2 g |
| Acide lactique | qsp pH 3,5 | | |
| Eau purifiée | qsp | | 100 g. |

## Revendications

**Revendications pour les Etats contractants suivants : AT, BE, CH, DE, FR, GB, IT, LI, LU, NL, SE**

1. Composés de formule :

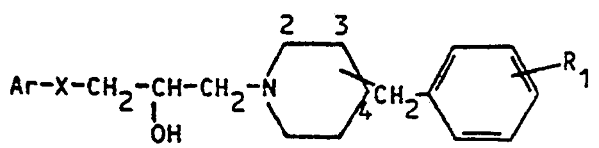

(I)

dans laquelle

— Ar représente un groupement phényle substitué par $R_2$, $R_3$ et $R_4$ ou un groupement naphtyle-1 ou naphtyle-2, lesdits groupements naphtyles étant non substitués ou substitués par 1 ou 2 atomes d'halogène ;

— X représente un atome d'oxygène ou un atome de soufre ;

— $R_1$ représente un atome d'hydrogène ou un atome d'halogène ;

— $R_2$ représente un atome d'halogène, un groupement trifluorométhyle, un groupement phényle non substitué ou substitué par 1 à 3 atomes d'halogène, un groupement benzyle non substitué ou substitué par 1 à 3 atomes d'halogène, ou un groupement phénoxy non substitué ou substitué par 1 à 3 atomes d'halogène, ou un groupe alkyle contenant de 1 à 4 atomes de carbone ;

— $R_3$ et $R_4$ représentent l'hydrogène, un atome d'halogène ou un groupe alkyle contenant de 1 à 4 atomes de carbone ;

— le groupement benzyle substitue le radical piperidino en position 2,3 ou 4 ;

ainsi que leurs sels avec des acides minéraux ou organiques.

2. Composés selon la revendication 1 de formule (I) dans laquelle le groupement Ar est le dichloro-2,4-phenyl.

3. (Benzyl-4 pipéridino)-1(dichloro-2,4 phénoxy)-3-propanol-2 et ses sels avec les acides minéraux ou organiques.

4. Méthanesulfonate de (benzyl-4 pipéridino)-1(dichloro-2,4 phénoxy)-3 propanol-2.

5. Procédé pour la préparation d'un composé selon l'une quelconque des revendications 1 à 4 caractérisé en ce que l'on traite l'époxyde de formule :

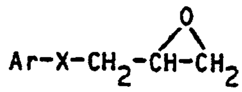

(II)

dans laquelle Ar et X ont les significations indiquées dans la revendication 1 par la benzyl pipéridine de formule:

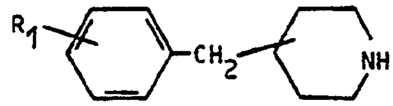

(III)

dans laquelle $R_1$ a la signification indiquée dans la revendication 1 et le groupement benzyle substitue la pipéridine en position 2, 3 ou 4, dans un solvant protique, à une température comprise entre la température ambiante et la température d'ébullition du solvant ; et l'on isole le composé selon la revendication 1 sous forme d'une base libre ou sous forme d'un sel avec un acide minéral ou organique.

6. Procédé selon la revendication 5, caractérisé en ce que le groupement Ar de l'époxyde de formule (II) est le dichloro-2,4 phényl.

7. Procédé selon l'une des revendications 5 ou 6, caractérisé en ce que l'on traite le (dichloro-2,4 phénoxy)-3 époxy-1,2 propane par la benzyl-4 pipéridine dans le méthanol à température ambiante et que l'on isole le (benzyl-4 pipéridino)-1 (dichloro-2,4 phénoxy)-3 propanol-2 obtenu.

8. Procédé selon la revendication 7, caractérisé en ce que le (benzyl-4 pipéridino)-1 (dichloro-2,4 phénoxy)-3 propanol-2 est isolé sous forme de méthanesulfonate.

9. Utilisation d'un composé selon l'une quelconque des revendications 1 à 4 comme antiseptique, désinfectant, conservateur, dans des préparations pharmaceutiques, désinfectantes (de surface), cosmétiques, vétérinaires ou alimentaires.

10. Composition pharmaceutique, caractérisée en ce qu'elle contient, à titre de principe actif, un composé selon l'une quelconque des revendications 1 à 4, en association avec un excipient pharmaceutiquement acceptable.

11. Composition pharmaceutique présentant une activité anti-microbienne et désinfectante, caractérisée en ce qu'elle contient de 0,01 à 5% d'un composé selon l'une quelconque des revendications 1 à 4.

12. Composition désinfectante pour surfaces inertes, caractérisée en ce qu'elle contient de 0,1 à 4% d'un composé selon l'une quelconque des revendications 1 à 4.

13. Composition pharmaceutique, caractérisée en ce qu'elle contient à titre de conservateur, de 0,005 à 0,5% d'un composé selon l'une quelconque des revendications 1 à 4.

14. Produit cosmétique caractérisé en ce qu'il contient à titre de conservateur de 0,005 à 0,5% d'un composé selon l'une quelconque des revendications 1 à 4.

15. Produit alimentaire caractérisé en ce qu'il contient à titre de conservateur de 0,005 à 0,5% d'un composé selon l'une quelconque des revendications 1 à 4.

**Revendications pour l'Etat contractant suivant : ES**

1. Procédé de préparation de composés de formule :

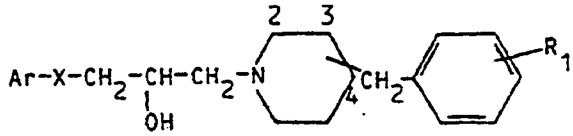

(I)

dans laquelle

— Ar représente un groupement phényle substitué par $R_2$, $R_3$ et $R_4$ ou un groupement naphtyle-1 ou naphtyle-2, lesdits groupements naphtyles étant non substitués ou substitués par 1 ou 2 atomes d'halogène ;

— X représente un atome d'oxygène ou un atome de soufre ;

— $R_1$ représente un atome d'hydrogène ou un atome d'halogène ;

— $R_2$ représente un atome d'halogène, un groupement trifluorométhyle, un groupement phényle non substitué ou substitué par 1 à 3 atomes d'halogène, un groupement benzyle non substitué ou substitué par 1 à 3 atomes d'halogène, ou un groupement phénoxy non substitué ou substitué par 1 à 3 atomes d'halogène, ou un groupe alkyle contenant de 1 à 4 atomes de carbone ;

— $R_3$ et $R_4$ représentent l'hydrogène, un atome d'halogène ou un groupe alkyle contenant de 1 à 4 atomes de carbone ;

— le groupement benzyle substitue le radical pipéridino en position 2, 3 ou 4 ;

ainsi que de leurs sels avec des acides minéraux ou organiques, caractérisé en ce qu'il consiste à traiter l'époxyde de formule :

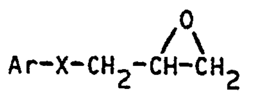

(II)

dans laquelle Ar et X ont les significations indiquées ci-dessus par la benzyl pipéridine de formule :

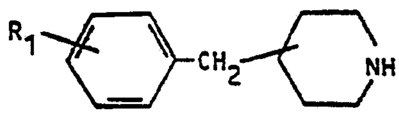

(III)

dans laquelle $R_1$ a la signification indiquée ci-dessus et le groupement benzyle substitue la pipéridine en position 2, 3 ou 4, dans un solvant protique, à une température comprise entre la température ambiante et la température d'ébullition du solvant ; et a isoler le composé de formule (I) obtenu sous forme d'un sel avec un acide minéral ou organique.

2. Procédé selon la revendication 1, caractérisé en ce que l'on traite l'époxyde de formule :

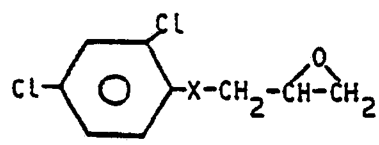

3. Procédé selon l'une quelconque des revendications 1 ou 2, caractérisé en ce que l'on traite le (dichloro-2,4 phénoxy)-3 époxy-1,2 propane par la benzyl-4 pipéridine dans le méthanol à température ambiante et l'on isole le (benzyl-4 pipéridino)-1 (dichloro-2,4 phénoxy)-3 propanol-2 obtenu.

4. Procédé selon la revendication 3, caractérisé en ce que le (benzyl-4 pipéridino)-1 (dichloro-2,4 phénoxy)-3 propanol-2 est isolé sous forme de méthanesulfonate.

5. Utilisation d'un composé de formule (I), obtenu selon l'une quelconque des revendications 1 à 4, comme antiseptique, désinfectant, conservateur, dans des préparations pharmaceutiques, désinfectantes (de surface), cosmétiques, vétérinaires ou alimentaires.

6. Utilisation d'un composé de formule (I), obtenu selon l'une quelconque des revendications 1 à 4, en association avec un véhicule pharmaceutiquement acceptable pour la préparation de compositions pharmaceutiques.

7. Utilisation selon la revendication 6, pour la préparation de compositions pharmaceutiques, présentant une activité anti-microbienne et désinfectante, contenant de 0,01 à 5% dudit composé de formule (I).

8. Composition désinfectante pour surfaces inertes, caractérisée en ce qu'elle contient de 0,1 à 4% d'un composé de formule (I), obtenu selon l'une quelconque des revendications 1 à 4.

9. Composition caractérisée en ce qu'elle contient, à titre de conservateur de 0,005 à 0,5% d'un composé de formule (I), obtenu selon l'une quelconque des revendications 1 à 4.

10. Produit cosmétique, caractérisé en ce qu'il contient à titre de conservateur de 0,005 à 0,5% d'un

composé de formule (I), obtenu selon l'une quelconque des revendications 1 à 4.

11. Produit alimentaire, caractérisé en ce qu'il contient à titre de conservateur de 0,005 à 0,5% d'un composé de formule (I), obtenu selon l'une quelconque des revendications 1 à 4.

**Revendications pour l'Etat contractant suivant : GR**

1. Composé de formule :

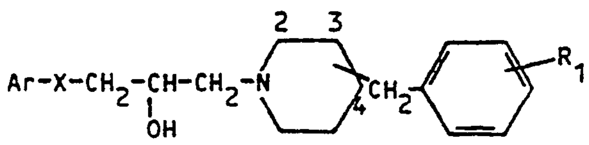

(I)

dans laquelle

— Ar représente un groupement phényle substitué par $R_2$, $R_3$ et $R_4$ ou un groupement naphtyle-1 ou naphtyle-2, lesdits groupements naphtyles étant non substitués ou substitués par 1 ou 2 atomes d'halogène ;

— X représente un atome d'oxygène ou un atome de soufre ;

— $R_1$ représente un atome d'hydrogène ou un atome d'halogène ;

— $R_2$ représente un atome d'halogène, un groupement trifluorométhyle, un groupement phényle non substitué ou substitué par 1 à 3 atomes d'halogène, un groupement benzyle non substitué ou substitué par 1 à 3 atomes d'halogène, ou un groupement phénoxy non substitué ou substitué par 1 à 3 atomes d'halogène, ou un groupe alkyle contenant de 1 à 4 atomes de carbone ;

— $R_3$ et $R_4$ représentent l'hydrogène, un atome d'halogène ou un groupe alkyle contenant de 1 à 4 atomes de carbone ;

— le groupement benzyle substitue le radical pipéridino en position 2, 3 ou 4 ;

ainsi que leurs sels avec des acides minéraux ou organiques.

2. Composés selon la revendicaiton 1 de formule (I) dans laquelle le groupement Ar est le dichloro-2,4 phényl.

3. (Benzyl-4 pipéridino)-1-(dichloro-2,4 phénoxy)-3- propanol-2 et ses sels avec les acides minéraux ou organiques.

4. Méthanesulfonate de (benzyl-4 pipéridino)-1(dichloro-2,4 phénoxy)-3 propanol-2.

5. Procédé pour la préparation d'un composé selon l'une quelconque des revendications 1 à 4 caractérisé en ce que l'on traite l'époxyde de formule :

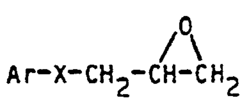

(II)

dans laquelle Ar et X ont les significations indiquées dans la revendication 1 par la benzyl pipéridine de formule:

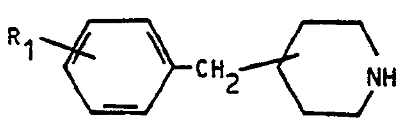

(III)

dans laquelle $R_1$ a la signification indiquée dans la revendication 1 et le groupement benzyle substitue la pipéridine en position 2, 3 ou 4, dans un solvant protique, à une température comprise entre la température ambiante et la température d'ébullition du solvant ; et l'on isole le composé selon le revendication 1 sous forme d'une base libre ou sous forme d'un sel avec un acide minéral ou organique.

6. Procédé selon la revendication 5, caractérisé en ce que le groupement Ar de l'époxyde de formule (II) est le dichloro-2,4 phényl.

7. Procédé selon l'une des revendications 5 ou 6, caractérisé en ce que l'on traite le (dichloro-2,4 phénoxy)-3 époxy-1,2 propane par la benzyl-4 pipéridine dans le méthanol à température ambiante et que l'on isole le (benzyl-4 pipéridino)-1 (dichloro-2,4 phénoxy)-3 propanol-2 obtenu.

8. Procédé selon la revendication 7, caractérisé en ce que le (benzyl-4 pipéridino)-1 (dichloro-2,4 phénoxy)-3 propanol-2 est isolé sous forme de méthanesulfonate.

9. Utilisation d'un composé selon l'une quelconque des revendications 1 à 4 comme antiseptique, désinfectant, conservateur, dans des préparations désinfectantes (de surface), cosmétiques, ou alimentaires.

10. Composition présentant une activité anti-microbienne, caractérisée en ce qu'elle contient de 0,01 à 5% d'un composé selon l'une quelconque des revendications 1 à 4.

11. Composition désinfectante pour surfaces inertes, caractérisée en ce qu'elle contient de 0,1 à 4% d'un composé selon l'une quelconque des revendications 1 à 4.

12. Composition caractérisée en ce qu'elle contient à titre de conservateur, de 0,005 à 0,5% d'un composé selon l'une quelconque des revendications 1 à 4.

13. Produit cosmétique caractérisé en ce qu'il contient à titre de conservateur de 0,005 à 0,5% d'un composé selon l'une quelconque des revendications 1 à 4.

14. Produit alimentaire caractérisé en ce qu'il contient à titre de conservateur de 0,005 à 0,5% d'un composé selon l'une quelconque des revendications 1 à 4.

## Patentansprüche

### Patentansprüche für folgende Vertragsstaaten : AT, BE, CH, DE, FR, GB, IT, LI, LU, NL, SE

1. Verbindung der Formel

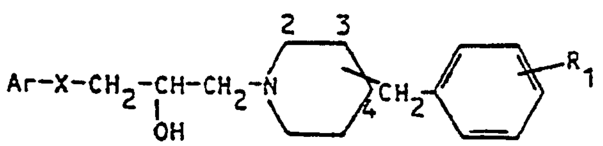

(I)

worin

— Ar eine durch $R_2$, $R_3$ und $R_4$ substituierte Phenylgruppe darstellt oder eine 1-Naphtyl- oder 2-Naphtylgruppe, wobei die genannten Naphtylgruppen unsubstituiert oder durch 1 oder 2 Halogenatome substituiert sind ;

— X ein Sauerstoffatom oder ein Schwefelatom darstellt ;

— $R_1$ ein Wasserstoffatom oder ein Halogenatom darstellt ;

— $R_2$ ein Halogenatom darstellt, eine Trifluormethylgruppe, eine unsubstituierte oder durch 1 bis 3 Halogenatome substituierte Phenylgruppe, eine unsubstituierte oder durch 1 bis 3 Halogenatome substituierte Benzylgruppe oder eine unsubstituierte oder durch 1 bis 3 Halogenatome substituierte Phenoxygruppe oder eine Alkylgruppe mit 1 bis 4 Kohlenstoffatomen ;

— $R_3$ und $R_4$ das Wasserstoff, ein Halogenatom oder eine Alkylgruppe mit 1 bis 4 Kohlenstoffatomen darstellt ;

— die Benzylgruppe das Piperidinradikal in 2, 3 oder 4 Stellung substituiert ;

sowie ihre Salze mit Mineralsäuren oder organischen Säuren.

2. Verbindung nach Anspruch 1 der Formel (I), in der die Gruppe Ar 2,4-Dichlorophenyl ist.

3. 1-(4-Benzylpiperidino)-3-(2,4-Dichlorophenoxy)-2-propanol und ihre Salze mit Mineralsäuren oder organischen Säuren.

4. 1-(4-Benzylpiperidino)-3-(2,4-Dichlorophenoxy)-2-propanol Methansulfonat.

5. Verfahren zur Herstellung einer Verbindung nach einem der Ansprüche 1 bis 4, dadurch gekennzeichnet, dass man das Epoxyd der Formel :

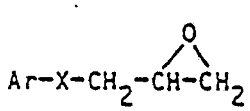

(II)

worin Ar und X die in Anspruch 1 angegebene Bedeutung haben, mit Benzylpiperidin der Formel :

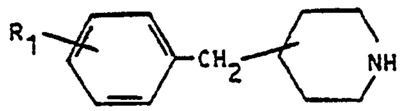

(III)

worin $R_1$ die in Anspruch 1 angegebene Bedeutung hat, und die Benzylgruppe das Piperidin in 2, 3 oder 4 Stellung substituiert, in einer protischen Losung bei einer zwischen Raumtemperatur und Siedetemperatur der Lösung liegenden Temperatur behandelt ; und man die Verbindung nach Anspruch 1 in Form einer freien Base oder in Form eines Salzes mit einer Mineralsäure oder organischen Säure isoliert.

6. Verfahren nach Anspruch 5, dadurch gekennzeichnet, dass die Ar-Gruppe des Epoxyds der Formel (II) 2,4-Dichlorophenyl ist.

7. Verfahren nach einem der Ansprüche 5 oder 6, dadurch gekennzeichnet, dass man das 3-(2,4-Dichlorophenoxy)-1,2-Epoxypropan mit 4-Benzylpiperidin in Methanol bei Raumtemperatur behandelt und dass man das erhaltene 1-(4-Benzylpiperidino)-3-(2,4-Dichlorophenoxy)-2-Propanol isoliert.

8. Verfahren nach Anspruch 7, dadurch gekennzeichnet, dass das 1-(4-Benzylpiperidino)-3-(2,4-Dichlorophenoxy)-2-Propanol in Form von Methansulfonat isoliert wird.

9. Verwendung einer Verbindung nach einem der Ansprüche 1 bis 4 als Antiseptikum, Desinfektionsmittel, Konservierungsmittel in pharmazeutischen, (Oberflächen-)desinfizierenden, kosmetischen, veterinären oder Nahrungsmittel- Zusammensetzungen.

10. Pharmazeutische Zusammensetzung, dadurch gekennzeichnet, dass sie eine Verbindung nach einem der Ansprüche 1 bis 4 als Wirkstoff in Verbindung mit einem pharmazeutisch akzeptablen Trägerstoff enthält.

11. Pharmazeutische Zusammensetzung mit antibakterieller und desinfizierender Aktivität, dadurch gekennzeichnet, dass sie 0,01 bis 5% einer Verbindung nach einem der Ansprüche 1 bis 4 enthält.

12. Desinfizierende Zusammensetzung für inerte Oberflächen, dadurch gekennzeichnet, dass sie 0,1 bis 4% einer Verbindung nach einem der Ansprüche 1 bis 4 enthält.

13. Pharmazeutische Zusammensetzung, dadurch gekennzeichnet, dass sie 0,005 bis 0,5% einer Verbindung nach einem der Ansprüche 1 bis 4 als Konservierungsmittel enthält.

14. Kosmetisches Mittel, dadurch gekennzeichnet, dass es 0,005 bis 0,5% einer Verbindung nach einem der Ansprüche 1 bis 4 als Konservierungsmittel enthält.

15. Nahrungsmittel, dadurch gekennzeichnet, dass es 0,005 bis 0,5% einer Verbindung nach einem der Ansprüche 1 bis 4 als Konservierungsmittel enthält.

**Patentansprüche für folgenden Vertragsstaat : ES**

1. Verfahren zur Herstellung einer Verbindung der Formel :

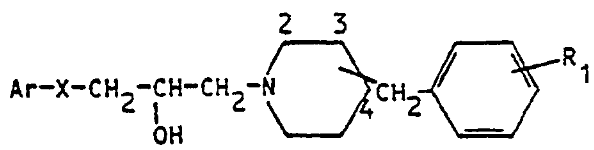

(I)

worin

— Ar eine durch $R_2$, $R_3$ und $R_4$ substituierte Phenylgruppe darstellt oder eine 1-Naphtyl- oder 2-Naphtylgruppe, wobei die genannten Naphtylgruppen unsubstituiert oder durch 1 oder 2 Halogenatome substituiert sind ;

— X ein Sauerstoffatom oder ein Schwefelatom darstellt ;

— $R_1$ ein Wasserstoffatom oder ein Halogenatom darstellt ;

— $R_2$ ein Halogenatom darstellt, eine Trifluormethylgruppe, eine unsubstituierte oder durch 1 bis 3 Halogenatome substituierte Phenylgruppe, eine unsubstituierte oder durch 1 bis 3 Halogenatome substituierte Benzylgruppe oder eine unsubstituierte oder durch 1 bis 3 Halogenatome substituierte Phenoxygruppe oder eine Alkylgruppe mit 1 bis 4 Kohlenstoffatomen ;

— $R_3$ und $R_4$ das Wasserstoff, ein Halogenatom oder eine Alkylgruppe mit 1 bis 4 Kohlenstoffatomen darstellt ;

— die Benzylgruppe das Piperidinradikal in 2, 3 oder 4 Stellung substituiert ;

sowie ihre Salze mit Mineralsäuren oder organischen Säuren, dadurch gekennzeichnet, dass es darin besteht, dass man das Epoxyd der Formel :

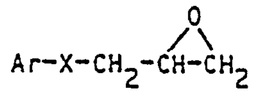

(II)

worin Ar und X die oben angegebene Bedeutung haben, mit Benzylpiperidin der Formel :

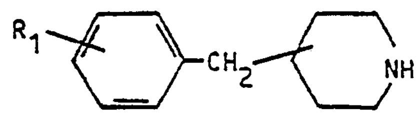

(III)

worin $R_1$ die oben angegebene Bedeutung hat, und die Benzylgruppe das Piperidin in 2, 3 oder 4 Stellung substituiert, in einer protischen Lösung bei einer zwischen Raumtemperatur und Siedetemperatur der Lösung liegenden Temperatur behandelt ; und man die in Form eines Salzes mit einer Mineralsäure oder organischen Säure erhaltenen Verbindung der Formel (I) isoliert.

2. Verfahren nach Anspruch 1, dadurch gekennzeichnet, dass man das Epoxyd der Formel :

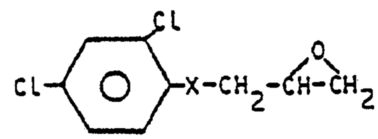

behandelt.

3. Verfahren nach einem der Ansprüche 1 oder 2, dadurch gekennzeichnet, dass man das 3-(2,4-Dichlorophenoxy)-1,2-Epoxypropan mit 4-Benzylpiperidin in Methanol bei Raumtemperatur behandelt und dass man das erhaltene 1-(4-Benzylpiperidino)-3-(2,4-Dichlorophenoxy)-2-Propanol isoliert.

4. Verfahren nach Anspruch 3, dadurch gekennzeichnet, dass das 1-(4-Benzylpiperidino)-3-(2,4-Dichlorophenoxy)-2-Propanol in Form von Methansulfonat isoliert wird.

5. Verwendung einer nach einem der Ansprüche 1 bis 4 erhaltenen Verbindung der Formel (I) als Antiseptikum, Desinfektionsmittel, Konservierungsmittel in pharmazeutischen, (Oberflächen-)desinfizierenden, kosmetischen, veterinären oder Nahrungsmittel- Zusammensetzungen.

6. Verwendung einer nach einem der Ansprüche 1 bis 4 erhaltenen Verbindung der Formel (I) zusammen mit einem pharmazeutisch akzeptablen Vehikel für die Herstellung von pharmazeutischen Zusammensetzungen.

7. Verwendung nach Anspruch 6, für die Herstellung von pharmazeutischen Zusammensetzungen mit antibakterileller und desinfizierender Aktivität, die 0,01 bis 5% der genannten Verbindung der Formel (I) enthalten.

8. Desinfizierende Zusammensetzung für inerte Oberflächen, dadurch gekennzeichnet, dass sie 0,1 bis 4% einer nach einem der Ansprüche 1 bis 4 erhaltenen Verbindung der Formel (I) enthält.

9. Zusammensetzung, dadurch gekennzeichnet, dass sie 0,005 bis 0,5% einer nach einem der Ansprüche 1 bis 4 erhaltenen Verbindung der Formel (I) als Konservierungsmittel enthält.

10. Kosmetisches Mittel, dadurch gekennzeichnet, dass es 0,005 bis 0,5% einer nach einem der Ansprüche 1 bis 4 erhaltenen Verbindung der Formel (I) als Konservierungsmittel enthält.

11. Nahrungsmittel, dadurch gekennzeichnet, dass es 0,005 bis 0,5% einer nach einem der Ansprüche 1 bis 4 erhaltenen Verbindung der Formel (I) als Konservierungsmittel enthält.

**Patentansprüche für folgenden Vertragsstaat : GR**

1. Verbindung der Formel

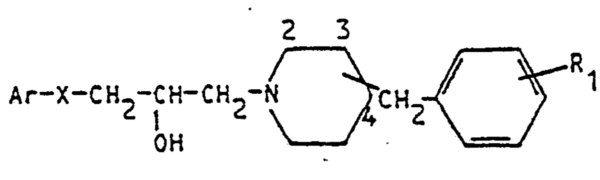

(I)

worin

— Ar eine durch $R_2$, $R_3$ und $R_4$ substituierte Phenylgruppe darstellt oder eine 1-Naphtyl- oder 2-Naphtylgruppe, wobei die genannten Naphtylgruppen unsubstituiert oder durch 1 oder 2 Halogenatome substituiert sind ;

— X ein Sauerstoffatom oder ein Schwefelatom darstellt ;

— $R_1$ ein Wasserstoffatom oder ein Halogenatom darstellt ;

— $R_2$ ein Halogenatom darstellt, eine Trifluormethylgruppe, eine unsubstituierte oder durch 1 bis 3 Halogenatome substituierte Phenylgruppe, eine unsubstituierte oder durch 1 bis 3 Halogenatome substituierte Benzylgruppe oder eine unsubstituierte oder durch 1 bis 3 Halogenatome substituierte Phenoxygruppe oder eine Alkylgruppe mit 1 bis 4 Kohlenstoffatomen ;

— $R_3$ und $R_4$ das Wasserstoff, ein Halogenatom oder eine Alkylgruppe mit 1 bis 4 Kohlenstoffatomen darstellt ;

— die Benzylgruppe das Piperidinradikal in 2, 3 oder 4 Stellung substituiert ;

sowie ihre Salze mit Mineralsäuren oder organischen Säuren.

2. Verbindung nach Anspruch 1 der Formel (I), in der die Gruppe Ar 2,4-Dichlorophenyl ist.

3. 1-(4-Benzylpiperidino)-3-(2,4-Dichlorophenoxy)-2-propanol und ihre Salze mit Mineralsäuren oder organischen Säuren.

4. 1-(4-Benzylpiperidino)-3-(2,4-Dichlorophenoxy)-2-propanol Methansulfonat.

5. Verfahren zur Herstellung einer Verbindung nach einem der Ansprüche 1 bis 4, dadurch gekennzeichnet, dass man das Epoxyd der Formel :

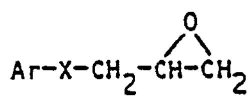

(II)

worin Ar und X die in Anspruch 1 angegebene Bedeutung haben, mit Benzylpiperidin der Formel :

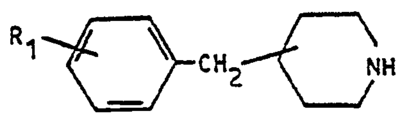

(III)

worin $R_1$ die in Anspruch 1 angegebene Bedeutung hat, und die Benzylgruppe das Piperidin in 2, 3 oder 4 Stellung substituiert, in einer protischen Lösung bei einer zwischen Raumtemperatur und Siedetemperatur der Lösung liegenden Temperatur behandelt ; und man die Verbindung nach Anspruch 1 in Form einer freien Base oder in Form eines Salzes mit einer Mineralsäure oder organischen Saure isoliert.

6. Verfahren nach Anspruch 5, dadurch gekennzeichnet, dass die Ar-Gruppe des Epoxyds der Formel (II) 2,4-Dichlorophenyl ist.

7. Verfahren nach einem der Ansprüche 5 oder 6, dadurch gekennzeichnet, dass man das 3-(2,4-Dichlorophenoxy)-1,2-Epoxypropan mit 4-Benzylpiperidin in Methanol bei Raumtemperatur behandelt und dass man das erhaltene 1-(4-Benzylpiperidino)-3-(2,4-Dichlorophenoxy)-2-Propanol isoliert.

8. Verfahren nach Anspruch 7, dadurch gekennzeichnet, dass das 1-(4-Benzylpiperidino)-3-(2,4-Dichlorophenoxy)-2-Propanol in Form von Methansulfonat isoliert wird.

9. Verwendung einer Verbindung nach einem der Ansprüche 1 bis 4 als Antiseptikum, Desinfektionsmittel, Konservierungsmittel in pharmazeutischen, (Oberflächen-)desinfizierenden, kosmetischen, veterinären oder Nahrungsmittel- Zusammensetzungen.

10. Zusammensetzung, dadurch gekennzeichnet, dass sie eine Verbindung nach einem der Ansprüche 1 bis 4 als Wirkstoff in Verbindung mit einem pharmazeutisch akzeptablen Trägerstoff enthält.

11. Desinfizierende Zusammensetzung für inerte Oberflächen, dadurch gekennzeichnet, dass sie 0,1 bis 4% einer Verbindung nach einem der Ansprüche 1 bis 4 enthält.

12. Pharmazeutische Zusammensetzung, dadurch gekennzeichnet, dass sie 0,005 bis 0,5% einer Verbindung nach einem der Ansprüche 1 bis 4 als Konservierungsmittel enthält.

13. Kosmetisches Mittel, dadurch gekennzeichnet, dass es 0,005 bis 0,5% einer Verbindung nach einem der Ansprüche 1 bis 4 als Konservierungsmittel enthält.

14. Nahrungsmittel, dadurch gekennzeichnet, dass es 0,005 bis 0,5% einer Verbindung nach einem der Ansprüche 1 bis 4 als Konservierungsmittel enthält.

## Claims

**Claims for the following contracting States : AT, BE, CH, DE, FR, GB, IT, LI, LU, NL, SE**

1. Compounds of the formula

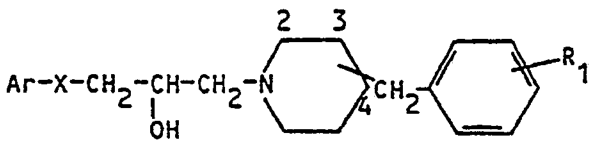

(I),

in which

— Ar represents a phenyl group substituted by $R_2$, $R_3$ and $R_4$, or a naphth-1-yl or naphth-2-yl group, the said naphthyl groups being unsubstituted or substituted by 1 or 2 halogen atoms ;
— X represents an oxygen atom or a sulfur atom ;
— $R_1$ represents a hydrogen atom or a halogen atom ;
— $R_2$ represents a halogen atom, a trifluoromethyl group, a phenyl group which is unsubstituted or substituted by 1 to 3 halogen atoms, a benzyl group which is unsubstituted or substituted by 1 to 3 halogen atoms, a phenoxy group which is unsubstituted or substituted by 1 to 3 halogen atoms, or an alkyl group containing from 1 to 4 carbon atoms ;
— $R_3$ and $R_4$ represent hydrogen, a halogen atom or an alkyl group containing from 1 to 4 carbon atoms; and
— the benzyl group substitutes the piperidino radical in the 2-, 3- or 4-position,

and their salts with mineral or organic acids.

2. Compounds according to claim 1 of formula (I) in which the group Ar is 2,4-dichlorophenyl.

3. 1-(4-Benzylpiperidino)-3-(2,4-dichlorophenoxy)propan-2-ol and its salts with mineral or organic acids.

4. 1-(4-Benzylpiperidino)-3-(2,4-dichlorophenoxy)propan-2-ol methanesulfonate.

5. Process for the preparation of a compound according to any one of claims 1 to 4, characterized in that it comprises treating the epoxide of the formula :

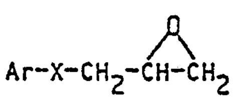

(II),

in which Ar and X have the meanings indicated in claim 1, with the benzylpiperidine of the formula :

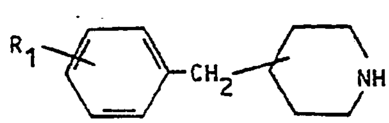

(III),

in which $R_1$ has the meaning indicated in claim 1 and the benzyl group substitutes the piperidine in the 2-,3-or 4-position, in a protic solvent, at a temperature between room temperature and the boiling point of the solvent, and isolating the compound according to claim 1 in the form of a free base or in the form of a salt with a mineral or organic acid.

6. Process according to claim 5, characterized in that the group Ar of the epoxide of formula (II) is 2,4-dichlorophenyl.

7. Process according to claim 5 or claim 6, characterized in that 3-(2,4-dichlorophenoxy)-1,2-epoxypropane is treated with 4-benzylpiperidine in methanol, at room temperature, and the 1-(4-benzylpiperidino)-3-(2,4-dichlorophenoxy)propan-2-ol obtained is isolated.

8. Process according to claim 7, characterized in that the 1-(4-benzylpiperidino)-3-(2,4-dichlorophenoxy)propan-2-ol is isolated in the form of the methanesulfonate.

9. Use of a compound according to any one of claims 1 to 4 as an antiseptic, disinfectant or preservative in pharmaceutical, (surface) disinfectant, cosmetic, veterinary or food preparations.

10. Pharmaceutical composition characterized in that it contains a compound according to any one of

claims 1 to 4 as the active principle, in association with a pharmaceutically acceptable excipient.

11. Pharmaceutical composition having an antimicrobial and disinfectant activity, characterized in that it contains from 0.01 to 5% of a compound according to any one of claims 1 to 4.

12. Disinfectant composition for inert surfaces, characterized in that it contains from 0.1 to 4% of a compound according to any one of claims 1 to 4.

13. Pharmaceutical composition characterized in that it contains from 0.005 to 0.5% of a compound according to any one of claims 1 to 4 as a preservative.

14. Cosmetic product characterized in that it contains from 0.005 to 0.5% of a compound according to any one of claims 1 to 4 as a preservative.

15. Food product characterized in that it contains from 0.005 to 0.5% of a compound according to any one of claims 1 to 4 as a preservative.

**Claims for the following contracting States : ES**

1. Process for the preparation of compounds of the formula :

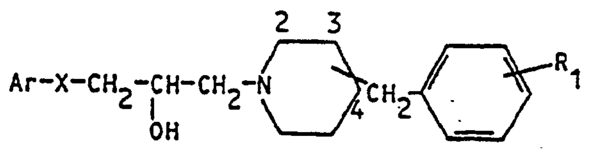
(I),

in which

— Ar represents a phenyl group substituted by $R_2$, $R_3$ and $R_4$, or a naphth-1-yl or naphth-2-yl group, the said naphthyl groups being unsubstituted or substituted by 1 or 2 halogen atoms ;

— X represents an oxygen atom or a sulfur atom ;

— $R_1$ represents a hydrogen atom or a halogen atom ;

— $R_2$ represents a halogen atom, a trifluoromethyl group, a phenyl group which is unsubstituted or substituted by 1 to 3 halogen atoms, a benzyl group which is unsubstituted or substituted by 1 to 3 halogen atoms, a phenoxy group which is unsubstituted or substituted by 1 to 3 halogen atoms, or an alkyl group containing from 1 to 4 carbon atoms ;

— $R_3$ and $R_4$ represent hydrogen, a halogen atom or an alkyl group containing from 1 to 4 carbon atoms; and

— the benzyl group substitutes the piperidino radical in the 2-, 3- or 4-position,

and their salts with mineral or organic acids, characterized in that it comprises treating the epoxide of the formula :

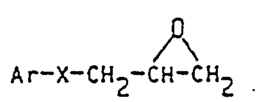
(II),

in which Ar and X have the meanings indicated above, with the benzylpiperidine of the formula :

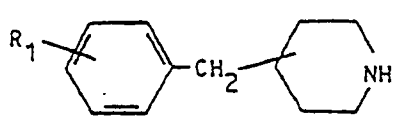
(III),

in which $R_1$ has the meaning indicated above and the benzyl group substitutes the piperidine in the 2-, 3- or 4-position, in a protic solvent, at a temperature between room temperature and the boiling point of the solvent, and isolating the obtained compound of formula (I) in the form of a free base or in the form of a salt with mineral or organic acid.

2. Process according to claim 1, characterized in that an epoxide of formula :

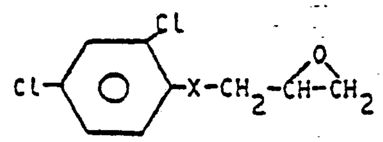

is treated.

3. Process according to any one of claims 1 or 2, characterized in that 3-(2,4-dichlorophenoxy)-1,2-epoxypropane is treated with 4-benzylpiperidine in methanol, at room temperature, and the 1-(4-benzylpiperidino)-3-(2,4-dichlorophenoxy)propan-2-ol obtained is isolated.

4. Process according to claim 3, wherein the 1-(4-benzylpiperidino)-3-(2,4-dichlorophenoxy)propan-2-ol is isolated in the form of the methanesulfonate.

5. Use of a compound of formula (I) obtained according to any one of claims 1 to 4 as an antiseptic, disinfectant or preservative in pharmaceutical, (surface) disinfectant, cosmetic, veterinary or food preparations.

6. Use of a compound of formula (I) obtained according to any one of claims 1 to 4 in association with a pharmaceutically acceptable excipient for the preparation of pharmaceutical compositions.

7. Use according to claim 6, for the preparation of pharmaceutical compositions having an antimicrobial and disinfectant activity, which contain from 0.01 to 5% of a compound of formula (I).

8. Disinfectant composition for inert surfaces, characterized in that it contains from 0.1 to 4% of a compound of formula (I), obtained according to any one of claims 1 to 4.

9. Composition characterized in that it contains, as a preservative, from 0.005 to 0.5% of a compound of formula (I) obtained according to any one of claims 1 to 4.

10. Cosmetic product characterized in that it contains, as a preservative, from 0.005 to 0.5% of a compound of formula (I) obtained according to any one of claims 1 to 4.

11. Food product characterized in that it contains, as a preservative, from 0.005 to 0.5% of a compound as of formula (I) obtained according to any one of claims 1 to 4.

**Claims for the following contracting State : GR**

1. Compounds of the formula

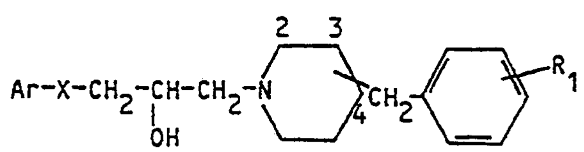

(I),

in which

- Ar represents a phenyl group substituted by $R_2$, $R_3$ and $R_4$, or a naphth-1-yl or naphth-2-yl group, the said naphthyl groups being unsubstituted or substituted by 1 or 2 halogen atoms ;
- X represents an oxygen atom or a sulfur atom ;
- $R_1$ represents a hydrogen atom or a halogen atom ;
- $R_2$ represents a halogen atom, a trifluoromethyl group, a phenyl group which is unsubstituted or substituted by 1 to 3 halogen atoms, a benzyl group which is unsubstituted or substituted by 1 to 3 halogen atoms, a phenoxy group which is unsubstituted or substituted by 1 to 3 halogen atoms, or an alkyl group containing from 1 to 4 carbon atoms ;
- $R_3$ and $R_4$ represent hydrogen, a halogen atom or an alkyl group containing from 1 to 4 carbon atoms; and
- the benzyl group substitutes the piperidino radical in the 2-, 3- or 4-position,

and their salts with mineral or organic acids.

2. Compounds according to claim 1 of formula (I) in which the group Ar is 2,4-dichlorophenyl.

3. 1-(4-Benzylpiperidino)-3-(2,4-dichlorophenoxy)propan-2-ol and its salts with mineral or organic acids.

4. 1-(4-Benzylpiperidino)-3-(2,4-dichlorophenoxy)propan-2-ol methanesulfonate.

5. Process for the preparation of a compound according to any one of claims 1 to 4, characterized in that it comprises treating the epoxide of the formula :

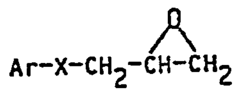 (II),

in which Ar and X have the meanings indicated in claim 1, with the benzylpiperidine of the formula :

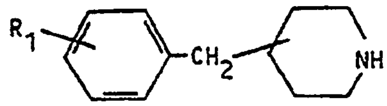 (III),

in which $R_1$ has the meaning indicated in claim 1 and the benzyl group substitutes the piperidine in the 2-,3- or 4-position, in a protic solvent, at a temperature between room temperature and the boiling point of the solvent, and isolating the compound according to claim 1 in the form of a free base or in the form of a salt with a mineral or organic acid.

6. Process according to claim 5, characterized in that the group Ar of the epoxide of formula (II) is 2,4-dichlorophenyl.

7. Process according to claim 5 or claim 6, characterized in that 3-(2,4-dichlorophenoxy)-1,2-epoxypropane is treated with 4-benzylpiperidine in methanol, at room temperature, and the 1-(4-benzylpiperidino)-3-(2,4-dichlorophenoxy)propan -2-ol obtained is isolated.

8. Process according to claim 7, characterized in that the 1-(4-benzylpiperidino)-3-(2,4-dichlorophenoxy)propan -2-ol is isolated in the form of the methanesulfonate.

9. Use of a compound according to any one of claims 1 to 4 as an antiseptic, disinfectant, preservative in (surface) disinfectant, cosmetic, or food preparations.

10. Composition having an antimicrobial activity, characterized in that it contains from 0.01 to 5% of a compound according to any one of claims 1 to 4.

11. Disinfectant composition for inert surfaces, characterized in that it contains from 0.1 to 4% of a compound according to any one of claims 1 to 4.

12. Composition characterized in that it contains from 0.005 to 0.5% of a compound according to any one of claims 1 to 4, as a preservative.

13. Cosmetic product characterized in that it contains from 0.005 to 0.5% of a compound according to any one of claims 1 to 4 as a preservative.

14. Food product characterized in that it contains from 0.005 to 0.5% of a compound according to any one of claims 1 to 4, as a preservative.